(19) Europäisches Patentamt
European Patent Office
Office européen des brevets

(11) Publication number: **0 637 588 A1**

# (12) EUROPEAN PATENT APPLICATION published in accordance with Art. 158(3) EPC

(21) Application number: **94904739.3**

(22) Date of filing: **19.01.94**

(86) International application number: **PCT/JP94/00067**

(87) International publication number: **WO 94/17077 (04.08.94 94/18)**

(51) Int. Cl.[6]: **C07F 1/08**, C07F 13/00, A01N 1/02

(30) Priority: **19.01.93 JP 6736/93**

(43) Date of publication of application: **08.02.95 Bulletin 95/06**

(84) Designated Contracting States: **AT BE CH DE DK ES FR GB GR IE IT LI LU MC NL PT SE**

(71) Applicant: **SHISEIDO COMPANY LIMITED**
**5-5 Ginza 7-chome**
**Chuo-ku**
**Tokyo 104 (JP)**

(72) Inventor: **NISHINO, Chikao, The Shiseido Research Center (2)**
**2-12-1, Fukuura**
**Kanazawa-ku**
**Yokohama-shi**
**Kanagawa2236 (JP)**
Inventor: **INADA, Ryuhei, The Shiseido Research Center (2)**
**2-12-1, Fukuura**
**Kanazawa-ku**
**Yokohama-shi**
**Kanagawa 236 (JP)**
Inventor: **NISHIKIORI, Kouji, The Shiseido Res. Center (2)**
**2-12-1, Fukuura**
**Kanazawa-ku**
**Yokohama-shi**
**Kanagawa 236 (JP)**
Inventor: **NAKASHIMA, Masaya, The Shiseido Res. Center (2)**
**2-12-1, Fukuura**
**Kanazawa-ku**
**Yokohama-shi**
**Kanagawa 236 (JP)**
Inventor: **EZURE, Tomonobu, The Shiseido Research Center (2)**
**2-12-1, Fukuura**
**anazawa-ku**
**Yokohama-shi**
**Kanagawa 236 (JP)**
Inventor: **NANBA, Ryujiro, The Shiseido Research Center (2)**
**2-12-1, Fukuura**
**Kanazawa-ku**
**Yokohama-shi**
**Kanagawa 236 (JP)**
Inventor: **KIKUCHI, Jun, The Shiseido Research Center (2)**
**2-12-1, Fukuura**
**Kanazawa-ku**
**Yokohama-shi**
**Kanagawa 236 (JP)**
Inventor: **KIMURA, Tomoko, The Shiseido Research Center (2)**
**2-12-1, Fukuura**
**Kanazawa-ku**
**Yokohama-shi**
**Kanagawa 236 (JP)**
Inventor: **KAJIKAWA, Yoshimi, Shiseido Company, Ltd.**
**Omori NM Building,**
**2-1-1, Omorikita**
**Ota-ku**
**Tokyo 143 (JP)**
Inventor: **WATANABE, Yuzuru, Shiseido Company, Ltd.**
**Omori NM Building,**
**2-1-1, Omorikita**
**Ota-ku**
**Tokyo 143 (JP)**

EP 0 637 588 A1

(74) Representative: **Henkel, Feiler, Hänzel & Partner**
**Möhlstrasse 37**
**D-81675 München (DE)**

(54) **ACTIVE OXYGEN INHIBITOR COMPOSITION.**

(57) An active oxygen inhibitor composition containing a coordinated complex represented by general formula (I) as the active ingredient (wherein $R_1$ and $R_2$ represent each independently hydrogen, $C_1$-$C_{12}$ alkyl, $C_1$-$C_{12}$ alkoxy, halogen, hydroxy, nitro, amino, carboxy or $C_2$-$C_6$ acyloxy; and M represents a central atom selected from among Cu, Mn and Zn); and a coordinated complex represented by general formula (II), wherein $R_{1-a}$ and $R_{2-a}$ are the same as the $R_1$ and $R_2$ defined in formula (I); and Ma represents either Cu or Mn, provided that the case where Ma represents Cu and each of $R_{1-a}$ and $R_{2-a}$ represents hydrogen, the case where $R_{1-a}$ represents hydrogen and $R_{2-a}$ represents hydroxy located either at the 4-position or at the 5-position, and the case where Ma represents Mn and each of $R_{1-a}$ and $R_{2-a}$ represents hydrogen are excepted.

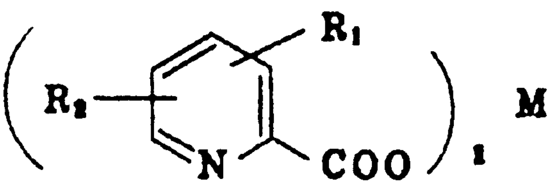

(I)

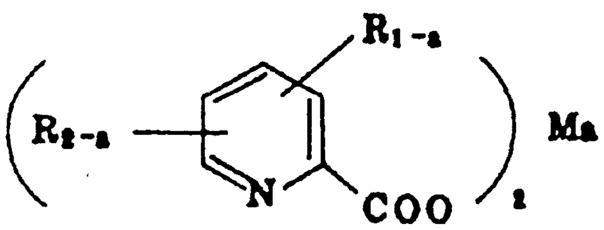

(II)

## TECHNICAL FIELD

The present invention relates to a coordinated complexes having a picolinic acid derivative as a ligand and an active oxygen suppressive composition containing the same as an effective ingredient. The active oxygen suppressing composition may be used to prevent or treat a disease arising from active oxygen in the body and to prevent various disorders arising due to active oxygen outside the body, especially is useful as a preservation solution of an organ.

## BACKGROUND ART

Along with the recent advances made in surgical techniques, immunosuppressive methods, etc., there has been an increase in the number of clinical cases of organ transplantation. When transplanting an organ, ideally the organ extracted from the donor is immediately transplanted into the recipient, but to enable effective utilization of precious organs for transplantation, it is necessary that organs are capable of being preserved for a long period. As methods for their preservation, there have been developed the method of low temperature preservation aimed mainly at suppression of metabolic action of the organ and the perfusion storage (or preservation method aimed mainly at maintaining the metabolic action.

Various proposals have been made as to the preservation solutions used for the above preservation methods, for example, as in Japanese Unexamined Patent Publication (Kokai) No. 1-246201.

Futhermore, generally speaking, the important matter regarding the composition of the preservation solution used in the low temperature preservation method is the capability to prevent edema in the tissue of organs to be transplanted and it is said that. The solution should contain effective ingredients so that 1) there is a suitable amount of substances hard to pass through the cell membrane and 2) the osmotic pressure is higher than that of plasma, 3) it is possible to prevent the occurrence of acidosis in the cell membranes, and 4) cell damage due to active oxygen is prevented. Japanese Unexamined Patent Publication (Kokai) No. 1-246201 in particular was directed at an improvement with respect to the above point 1), but glutathione is used for the purpose of 4). As other substances besides glutathione used for the purpose of 4), it is known in the field of art to use allopurinol, superoxide dismutase (SOD), $MgSO_4$, etc. In any case, it may be said, however, that these studies have just all been started. Further knowhow needs to be built up in this area.

## DISCLOSURE OF THE INVENTION

According, the object of the present invention is to provide an active oxygen suppressive composition which may be used for the prevention of various disorders due to active oxygen present at the outside of the body, such as enabling the prevention of cell disorders due to active oxygen in preservation solutions for organs, and for the prevention or treatment of various diseases arising from active oxygen in the body.

In accordance with the present invention, there is provided an active oxygen suppressive composition comprising as an effective ingredient, a coordinated complex, having the formula (I) containing a picolinic acid derivative as a ligand, and a carrier.

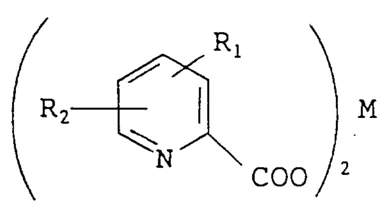

(I)

wherein, $R_1$ and $R_2$ independently represent a hydrogen atom, an alkyl group having 1 to 12 carbon atoms, an alkyloxy group having 1 to 12 carbon atoms, a halogen atom, a hydroxyl group, a nitro group, an amino group, a carboxyl group, or a lower acyloxy group having 2 to 6 carbon atoms and M represents a center atom selected from Cu, Mn, and Zn.

In accordance with the present invention, there is also provided a novel coordinated complex represented by the formula (II) which are included in the compounds shown in the formula (I) which may be used as the effective ingredient for the above-mentioned active oxygen suppressive composition:

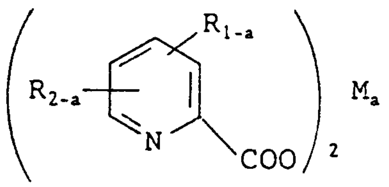

(II)

wherein, $R_{1-a}$ and $R_{2-a}$ have the same definitions as defined for $R_1$ and $R_2$ in the formula (I) and $M_a$ is Cu or Mn, provided that when $M_a$ is Cu and $R_{1-a}$ and $R_{2-a}$ are not simultaneously a hydrogen atom, when $R_{1-a}$ is a hydrogen atom and $R_{2-a}$ is not a hydroxyl group at the 4- or 5-position, and when $M_a$ is Mn, $R_{1-a}$ and $R_{2-a}$ are not simultaneously a hydrogen atom.

Best Mode For Carrying Out The Invention

The coordinated complexes able to be used for the object of the present invention have not been confirmed as to the type of their bonds, but it may be deduced that they have the structure shown by the following formula:

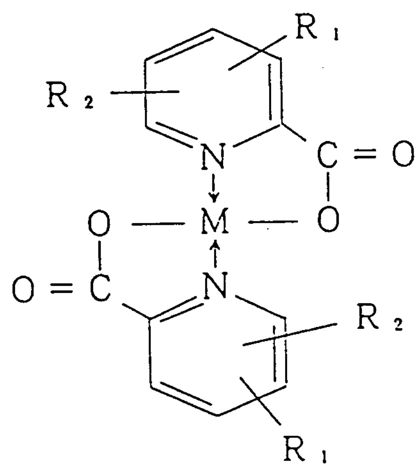

Regarding the positions of the substituent groups $R_1$ and $R_2$ in the above formula (I), so long as there is no adverse effect on the formation of the corresponding coordinated complexes, the substitution may be performed at any of the 3- to 6-positions of the picolinic acid ring, but in general in the case of a long chain alkyl, long chain alkoxyl, and nitro group, it is preferable that the substitution be performed at the 4-position.

The alkyl group usable as the substituent group includes alkyl groups having 1 to 12 carbon atoms, for example, methyl, ethyl, propyl, isopropyl, butyl, isobutyl, sec-butyl, tert-butyl, pentyl, hexyl, cyclopropyl, cyclobutyl, cyclohexyl, nonyl, decyl, undecyl, and dodecyl. Among these, methyl, ethyl, propyl, and isopropyl are preferable.

The alkyloxy group usable as the substituent group is a group derived from the substitution of the above alkyl group with an oxygen atom. For example, mention may be made of a methoxy, ethoxy, propoxy, isopropoxy, butoxy, tert-butoxy, cyclohexyloxy, decyloxy, and dodecyloxy group. In particular, a methoxy, ethoxy, propoxy, and isopropoxy group are preferable.

For the lower acyloxy group usable as the substituent group, mention may be made of acyloxy-groups having 1 to 6 carbon atoms, for example, an acetyloxy, propionyloxy, butyryloxy, tert-butyryloxy, and pentyloxy group. In particular, a methoxycarbonyl and ethoxycarbonyl group are preferable.

As other substituent groups, halogen atoms, mention may be made of chlorine, bromine, fluorine, iodine. Chlorine and fluorine are preferred. Further, the hydroxyl group, nitro group, and amino group may also be used as the substituent group R of the formula (I).

As the center atom M of the coordinated complexes of the formula (I), mention may be made of Cu, Mn, or Zn. Of these, ones comprised of Cu are particularly preferred.

The coordinated complexes specified by the above substituent groups and center atoms and used in the present invention may comprises any combinations of the same, but the specific combinations of

substituent groups, positions of substituent, and center metals mentioned in the following Table I are preferred.

Table I

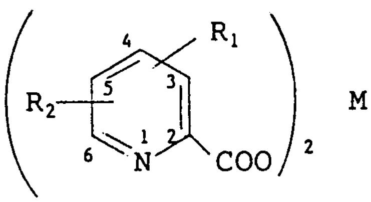

| Compound No. | $R_1$ | $R_2$ | Position of substituent | M |
|---|---|---|---|---|
| 1 | H | H | - | Cu |
| 2 | OH | H | 3 | Cu |
| 3 | OH | H | 4 | Cu |
| 4 | OH | H | 5 | Cu |
| 5 | OH | H | 6 | Cu |
| 6 | $OCH_3$ | H | 3 | Cu |
| 7 | $OC_2H_5$ | H | 3 | Cu |
| 8 | $OC_2H_5$ | H | 4 | Cu |
| 9 | $OC_2H_5$ | H | 5 | Cu |
| 10 | $OC_2H_5$ | H | 6 | Cu |
| 11 | $OC_3H_7$ | H | 3 | Cu |
| 12 | $OC_3H_7$ | H | 4 | Cu |
| 13 | $OC_3H_7$ | H | 5 | Cu |
| 14 | O(iso-$C_3H_7$) | H | 3 | Cu |
| 15 | O(iso-$C_3H_7$) | H | 4 | Cu |
| 16 | $OC_4H_9$ | H | 3 | Cu |
| 17 | $OC_4H_9$ | H | 4 | Cu |
| 18 | $OC_4H_9$ | H | 5 | Cu |
| 19 | O(tert $C_4H_9$) | H | 4 | Cu |
| 20 | $OC_5H_{11}$ | H | 3 | Cu |
| 21 | $OC_5H_{11}$ | H | 4 | Cu |

Table I (Continued)

| Compound No. | $R_1$ | $R_2$ | Position of substituent | M |
|---|---|---|---|---|
| 22 | $OC_5H_{11}$ | H | 5 | Cu |
| 23 | $OC_6H_{13}$ | H | 3 | Cu |
| 24 | $OC_6H_{13}$ | H | 4 | Cu |
| 25 | $OC_6H_{13}$ | H | 5 | Cu |
| 26 | 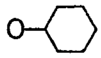 | H | 4 | Cu |
| 27 | $OC_7H_{15}$ | H | 3 | Cu |
| 28 | $OC_7H_{15}$ | H | 4 | Cu |
| 29 | $OC_8H_{17}$ | H | 3 | Cu |
| 30 | $OC_8H_{17}$ | H | 4 | Cu |
| 31 | $OC_9H_{19}$ | H | 3 | Cu |
| 32 | $OC_9H_{19}$ | H | 4 | Cu |
| 33 | $OC_{10}H_{21}$ | H | 3 | Cu |
| 34 | $OC_{10}H_{21}$ | H | 4 | Cu |
| 35 | $OC_{10}H_{21}$ | H | 5 | Cu |
| 36 | $OC_{10}H_{21}$ | H | 3 | Cu |
| 37 | $OC_{10}H_{21}$ | H | 4 | Cu |
| 38 | $OC_{10}H_{21}$ | H | 5 | Cu |
| 39 | $OC_{11}H_{23}$ | H | 3 | Cu |
| 40 | $OC_{11}H_{23}$ | H | 4 | Cu |
| 41 | $OC_{11}H_{23}$ | H | 5 | Cu |
| 42 | $OC_{12}H_{25}$ | H | 3 | Cu |
| 43 | $OC_{12}H_{25}$ | H | 4 | Cu |
| 44 | $OC_{12}H_{25}$ | H | 5 | Cu |
| 45 | $CH_3$ | H | 3 | Cu |

Table I (Continued)

| Compound No. | $R_1$ | $R_2$ | Position of substituent | M |
|---|---|---|---|---|
| 46 | $CH_3$ | H | 4 | Cu |
| 47 | $CH_3$ | H | 5 | Cu |
| 48 | $CH_3$ | H | 6 | Cu |
| 49 | $C_2H_5$ | H | 3 | Cu |
| 50 | $C_2H_5$ | H | 4 | Cu |
| 51 | $C_2H_5$ | H | 5 | Cu |
| 52 | $C_2H_5$ | H | 6 | Cu |
| 53 | $C_3H_5$ | H | 3 | Cu |
| 54 | $C_3H_5$ | H | 4 | Cu |
| 55 | $C_3H_5$ | H | 5 | Cu |
| 56 | $C_3H_5$ | H | 6 | Cu |
| 57 | iso-$C_3H_7$ | H | 3 | Cu |
| 58 | iso-$C_3H_7$ | H | 4 | Cu |
| 59 | iso-$C_3H_7$ | H | 5 | Cu |
| 60 | iso-$C_3H_7$ | H | 6 | Cu |
| 61 | $C_4H_9$ | H | 3 | Cu |
| 62 | $C_4H_9$ | H | 4 | Cu |
| 63 | $C_4H_9$ | H | 5 | Cu |
| 64 | $C_4H_9$ | H | 6 | Cu |
| 65 | sec-$C_4H_9$ | H | 3 | Cu |
| 66 | sec-$C_4H_9$ | H | 4 | Cu |
| 67 | tert-$C_4H_9$ | H | 4 | Cu |
| 68 | $C_5H_{11}$ | H | 3 | Cu |
| 69 | $C_5H_{11}$ | H | 4 | Cu |

Table I (Continued)

| Compound No. | $R_1$ | $R_2$ | Position of substituent | M |
|---|---|---|---|---|
| 70 | $C_5H_{11}$ | H | 5 | Cu |
| 71 | $C_5H_{11}$ | H | 6 | Cu |
| 72 | $C_6H_{13}$ | H | 3 | Cu |
| 73 | $C_6H_{13}$ | H | 4 | Cu |
| 74 | $C_6H_{13}$ | H | 5 | Cu |
| 75 | 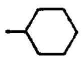 | H | 4 | Cu |
| 76 | $C_7H_{15}$ | H | 3 | Cu |
| 77 | $C_7H_{15}$ | H | 4 | Cu |
| 78 | $C_7H_{15}$ | H | 5 | Cu |
| 79 | $C_8H_{17}$ | H | 3 | Cu |
| 80 | $C_8H_{17}$ | H | 4 | Cu |
| 81 | $C_8H_{17}$ | H | 5 | Cu |
| 82 | $C_9H_{19}$ | H | 3 | Cu |
| 83 | $C_9H_{19}$ | H | 4 | Cu |
| 84 | $C_9H_{19}$ | H | 5 | Cu |
| 85 | $C_{10}H_{21}$ | H | 3 | Cu |
| 86 | $C_{10}H_{21}$ | H | 4 | Cu |
| 87 | $C_{10}H_{21}$ | H | 5 | Cu |
| 88 | $C_{11}H_{23}$ | H | 3 | Cu |
| 89 | $C_{11}H_{23}$ | H | 4 | Cu |
| 90 | $C_{11}H_{23}$ | H | 5 | Cu |
| 91 | $C_{12}H_{25}$ | H | 3 | Cu |
| 92 | $C_{12}H_{25}$ | H | 4 | Cu |
| 93 | Cl | H | 3 | Cu |

Table I (Continued)

| Compound No. | $R_1$ | $R_2$ | Position of substituent | M |
|---|---|---|---|---|
| 94 | Cl | H | 4 | Cu |
| 95 | Cl | H | 5 | Cu |
| 96 | Cl | H | 6 | Cu |
| 97 | F | H | 4 | Cu |
| 98 | F | H | 6 | Cu |
| 99 | $NO_2$ | H | 4 | Cu |
| 100 | COOH | H | 4 | Cu |
| 101 | $NH_2$ | H | 4 | Cu |
| 102 | $NH_2$ | H | 6 | Cu |
| 103 | $OCOCH_3$ | H | 3 | Cu |
| 104 | $OCOCH_3$ | H | 4 | Cu |
| 105 | $OCOCH_3$ | H | 5 | Cu |
| 106 | $OCOCH_3$ | H | 6 | Cu |
| 107 | $OCOC_2H_5$ | H | 3 | Cu |
| 108 | $OCOC_2H_5$ | H | 4 | Cu |
| 109 | $OCOC_2H_5$ | H | 5 | Cu |
| 110 | $OCOC_2H_5$ | H | 6 | Cu |
| 111 | $OCOC_3H_7$ | H | 3 | Cu |
| 112 | $OCOC_3H_7$ | H | 4 | Cu |
| 113 | $OCOC_3H_7$ | H | 5 | Cu |
| 114 | $OCOC_4H_9$ | H | 3 | Cu |
| 115 | $OCOC_4H_9$ | H | 4 | Cu |
| 116 | $OCOC_5H_{11}$ | H | 4 | Cu |

Table I (Continued)

| Compound No. | $R_1$ | $R_2$ | Position of substituent ($R_1$, $R_2$) | M |
|---|---|---|---|---|
| 117 | OH | OH | 3, 5 | Cu |
| 118 | OH | OH | 4, 6 | Cu |
| 119 | OH | OH | 3, 6 | Cu |
| 120 | OH | $CH_3$ | 5, 4 | Cu |
| 121 | OH | $C_6H_{13}$ | 5, 4 | Cu |
| 122 | OH | $C_{12}H_{25}$ | 5, 4 | Cu |
| 123 | $OCH_3$ | $OCH_3$ | 3, 5 | Cu |
| 124 | $OCH_3$ | $OCH_3$ | 3, 6 | Cu |
| 125 | $OC_6H_{13}$ | $C_6H_{13}$ | 3, 5 | Cu |
| 126 | $OC_6H_{13}$ | $C_6H_{13}$ | 3, 6 | Cu |
| 127 | $CH_3$ | $CH_3$ | 4, 5 | Cu |
| 128 | $C_6H_{13}$ | $C_6H_{13}$ | 4, 6 | Cu |
| 129 | $C_{12}H_{25}$ | $C_{12}H_{25}$ | 4, 6 | Cu |
| 130 | Cl | Cl | 3, 5 | Cu |
| 131 | Cl | Cl | 4, 6 | Cu |
| 132 | Cl | Cl | 3, 6 | Cu |
| 133 | F | F | 4, 6 | Cu |
| 134 | $NO_2$ | $NO_2$ | 4, 6 | Cu |
| 135 | $NH_2$ | $NH_2$ | 4, 6 | Cu |
| 136 | $CH_3$ | $OCH_3$ | 4, 5 | Cu |
| 137 | $C_6H_{13}$ | $OCH_3$ | 4, 5 | Cu |
| 138 | $C_{12}H_{25}$ | $OCH_3$ | 4, 5 | Cu |
| 139 | $CH_3$ | $OC_6H_{13}$ | 4, 5 | Cu |
| 140 | $C_6H_{13}$ | $OC_6H_{13}$ | 4, 5 | Cu |

Table I (Continued)

| Compound No. | $R_1$ | $R_2$ | Position of substituent ($R_1$, $R_2$) | M |
|---|---|---|---|---|
| 141 | $C_{12}H_{25}$ | $OC_6H_{13}$ | 4, 5 | Cu |
| 142 | $CH_3$ | $OC_{12}H_{25}$ | 4, 5 | Cu |
| 143 | $C_6H_{13}$ | $OC_{12}H_{25}$ | 4, 5 | Cu |
| 144 | $C_{12}H_{25}$ | $OC_{12}H_{25}$ | 4, 5 | Cu |
| 145 | $CH_3$ | Cl | 4, 5 | Cu |
| 146 | $C_6H_{13}$ | Cl | 4, 5 | Cu |
| 147 | $C_{12}H_{25}$ | Cl | 4, 5 | Cu |
| 148 | $CH_3$ | F | 4, 5 | Cu |
| 149 | $C_6H_{13}$ | F | 4, 5 | Cu |
| 150 | $C_{12}H_{25}$ | F | 4, 5 | Cu |
| 151 | $CH_3$ | $NO_2$ | 4, 5 | Cu |
| 152 | $C_6H_7$ | $NO_2$ | 4, 5 | Cu |
| 153 | $C_{12}H_{25}$ | $NO_2$ | 4, 5 | Cu |
| 154 | $CH_3$ | $NH_2$ | 4, 6 | Cu |
| 155 | $C_6H_7$ | $NH_2$ | 4, 6 | Cu |
| 156 | $C_{12}H_{25}$ | $NH_2$ | 4, 6 | Cu |

Further, mention may be made, as other specific examples, the Compound Nos. 157 to 312 corresponding to the Compound Nos. 1 to 156 having the center metal Cu substituted with Mn and the Compound Nos. 313 to 468 corresponding to the Compound Nos. 1 to 156 having the Cu substituted with Zn.

The coordinated complexes of the Compound Nos. 1, 3, 4, and 157 are known compounds. The compounds other than these can be prepared in the same manner as the methods of production of these known compounds (Aliev, Z.G. et al., Izv. Akad. Nauk SSSR, Ser, Khim., (11), 2495 - 2501, 1988 and Kaneda, A. et al., Doshisha Daigaku Rikogaku Kenkyu Hokoku, 7 (4), 172 - 192, 1967).

For example, various compounds with various picolinic acid derivatives as bidentate ligands are known and some of these are even available commercially. Therefore, it is possible to make direct use of commercially available coordinated complexes, but it is also possible to obtain the targeted coordinated complexes by converting to the desired derivatives in accordance with known methods in the field of organic synthesis, then performing a chelating reaction with salts of Cu, Mn, or Zn. The chelating reaction usually may be easily performed by heating a metal salt and a picolinic acid derivative of about 2 times the amount of the same in an aqueous solution adjusted to an alkaline condition, if necessary. These reactions proceed in a good yield. The chelated product (coordinated complex) may be easily isolated since it is generally insoluble in aqueous solvents compared with the starting materials. The metal salt used may be any one so long as it is soluble in water, but for Cu and Zn, use of their acetates is preferable. Further, for Mn, the chloride of the same can be used. The coordinated complexes thus obtained may be purified by, for example, recrystallization, if necessary, and used for the objects of the present invention.

The coordinated complexes having the formula (I) having picolinic acid derivatives as ligands according to the present invention are preferably used as, for example, preservation solutions for organs as active oxygen suppressive compositions. These compositions preferably contain 10 to 1500 μm, per weight of the composition, of the coordinated complexes having the formula (I), as effective ingredients, and further

contain general carriers. As the carriers, mention may be made of an electrolyte ($Na^+$, $K^+$, $Cl^-$, etc.), phosphate buffer, osmotic pressure adjuster (sugar), bactericide, etc. in an aqueous solution.

The active oxygen suppressive compositions according to the present invention may be prepared as liquids by mixing the coordinated complexes and carriers by a general method. Further, the coordinated complexes of the formula (I) having picolinic acid derivatives as ligands do not have toxicity or present any practical problems which would hinder use as liver storage solutions etc.

The specific examples of use of the active oxygen suppressive compositions according to the present invention will be explained below with reference to a preservation solution for organs of the cold perfusion storage method, but the present invention is not limited to these examples of use.

The coordinated complexes of the present invention may be used by adding the same to known preservation solutions for organ transplantation or by replacing parts thereof with the same. As a known preservation solution for organs, mention may be made of the Euro-Collins (EC) solution according to Squifflet J.P. et al. and as a typical solution for the preservation of the pancreas and liver, mention may be made of the Wisconsin (UW) solution of Wahlberg, J.A. et al. For example, see Squifflet, J.P. et al., Transplant. Proc., 13, 693, 1981 and Wahlberg, J.A., et al., Transplantation, 43, 5-8, 1987.

The specific composition of the EC solution is as follows:

| EC Solution | |
|---|---|
| Glucose | 194 mM |
| $K_2HPO_4$ | 42 mM |
| $KH_2PO_4$ | 15 mM |
| $NaHCO_3$ | 10 mM |
| KCl | 15 mM |
| pH 7.2, osmotic pressure 355 mOsm/L | |

The specific composition of the UW solution is as follows:

| UW Solution | |
|---|---|
| $K^+$ lactobionate | 100 mM |
| Raffinose | 30 mM |
| Hydroxyethyl starch | 50 g/L |
| $KH_2PO_4$ | 25 mM |
| $MgSO_4$ | 5 mM |
| Adenosine | 5 mM |
| Glutathione | 3 mM |
| Allopurinol | 1 mM |
| Insulin | 100 U/L |
| Dexamethasone | 8 mg/L |
| Bactrin* | 0.5 ml/L |
| pH 7.4, osmotic pressure 310 to 330 mOsm/L | |

* Bactrin: Mixed solution of trimethoprim (16 mg/ml) and sulfamethoxazole (80 mg/ml).

## EXAMPLES

The present invention will now be further explained in further detail by, but is by no means limited to, the following Examples.

Example 1 (Synthesis Example) Synthesis of Copper-Bis[3-Propoxy-2-Pyridinecarboxylate-$N^1$, $O^2$]

(Compound 11)

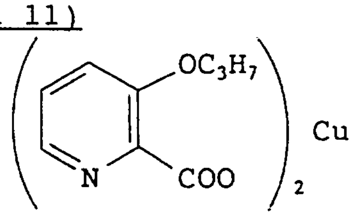

(a) Synthesis of 3-Propoxypicolinic acid propyl ether

A 2.8 g (0.02 mol) amount of 3-hydroxypicolinic acid and 3.7 g (0.02 mol) of propyl iodide were dissolved in 30 ml of dimethylformamide. To this was added 2.8 g (0.02 mol) of anhydrous potassium carbonate, then the mixture was heated and stirred at 60 °C for 1 hour and further at 100 °C for 1 hour. After cooling, 120 ml of ice water was added and extraction was performed with ethyl acetate. The solvent was distilled off under reduced pressure and separation was performed by silica gel column chromatography (ethyl acetate:hexane = 3:7) to obtain 1.00 g of a colorless oily substance. (Yield: 22.3%)

| | |
|---|---|
| GC-MS: | $M^+$223 |
| $^1$H-NMR (400 MHz)($CDCl_3$-$d_1$): | δ1.047 (dt, 6H), δ1.83 (m, 4dH), δ4.10 (t, 2H), δ4.32 (t, 2H), δ7.31 (dd, 1H), δ7.35 (dd, 1H), δ8.24 (dd, 1H) |

(b) Synthesis of 3-Propoxypicolinic Acid

A 1.8 g amount of 3-propoxypicolinic acid propyl ether was dissolved in 20 ml of methanol, then 20 ml of a 5% sodium hydroxide solution was added and the mixture was refluxed for 45 minutes. The solvent was distilled off under reduced pressure and the residue was dissolved in 10 ml of water. This was adjusted to a pH of about 2 by 6N hydrochloric acid, then was extracted well with ethyl acetate. This was dried with anhydrous sodium sulfate, then the solvent was distilled off and the remainder was recrystallized from ethyl acetate to obtain 0.92 g of a crystal. (Yield: 63.0%)

| | |
|---|---|
| m.p.: | 117.5 to 119 °C |
| GC-MS: | 137 ($M^+$-$CO_2$) |
| IR (KBr): | 1850, 1700, 1575 $cm^{-1}$ |

Table II

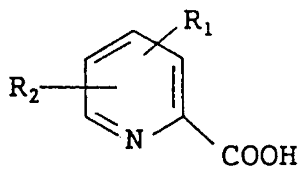

(III) ... Starting material

* Numbers show the position of substituent.

| Synthesis Example | Starting material R₁ | Starting material R₂ | Metal salt | Product (compound No.) (yield) m.p., IR (KBr) |
|---|---|---|---|---|
| 2 | H | H | Copper acetate | Compound 1 (95.2%) 300°C or more (decomposition), 1645, 1605, 1572, 1350 $cm^{-1}$ |
| 3 | OH(3)* | H | Copper acetate | Compound 2 (98.9%) 250°C or more (decomposition), 1649, 1611, 1570, 1329, 1248 $cm^{-1}$ |
| 4 | OH(4) | H | Copper acetate | Compound 3 (94.0%) 280°C or more (decomposition), 3254, 1663, 1615, 1339, 1260 $cm^{-1}$ |
| 5 | OH(5) | H | Copper acetate | Compound 4 (84.6%) 300°C or more (unknown), 1611, 1568, 1510, 1373, 1248 $cm^{-1}$ |
| 6 | OH(6) | H | Copper acetate | Compound 5 (84.4%) 300°C or more (decomposition), 1669, 1618, 1576, 1395, 1321, 1256 $cm^{-1}$ |
| 7 | $OCH_3$(2) | H | Copper acetate | Compound 6 (51.3%) 250°C or more (decomposition), 2948, 2843, 1671, 1636, 1580, 1289, 1242 $cm^{-1}$ |

Table II (Continued)

| Synthesis Example | Starting material R1 | R2 | Metal salt | Product (compound No.) (yield) m.p., IR (KBr) |
|---|---|---|---|---|
| 8 | $CH_3$(3) | H | Copper acetate | Compound 45 (88.2%)<br>270°C or more (decomposition), 2934, 1642, 1588, 1346 $cm^{-1}$ |
| 9 | $CH_3$(6) | H | Copper acetate | Compound 48 (62.8%)<br>260°C or more (decomposition), 1649, 1640, 1605, 1368, 1262 $cm^{-1}$ |
| 10 | $C_4H_9$(5) | H | Copper acetate | Compound 63 (81.9%)<br>250°C or more (decomposition), 2932, 2865, 1655, 1605, 1578, 1345 $cm^{-1}$ |
| 11 | Cl(4) | H | Copper acetate | Compound 94 (93.7%)<br>220°C or more (decomposition), 1651, 1595, 1559, 1341, 1101 $cm^{-1}$ |
| 12 | $NO_2$(4) | H | Copper acetate | Compound 99 (83.4%)<br>260°C or more (decomposition), 1657, 1599, 1545, 1354, 1341, 839 $cm^{-1}$ |
| 13 | COOH(4) | H | Copper acetate | Compound 100 (81.3%)<br>230°C or more (decomposition), 2793, 2508, 1726, 1638, 1613, 1564, 1368, 1287 $cm^{-1}$ |
| 14 | H | H | Manganese chloride | Compound 158 (50.0%)<br>235°C or more (decomposition), 1620, 1591, 1566, 1389 $cm^{-1}$ |

Table II (Continued)

| Synthesis Example | Starting material R1 | R2 | Metal salt | Product (compound No.) (yield) m.p., IR (KBr) |
|---|---|---|---|---|
| 15 | OH(3) | H | Zinc acetate | Compound 316 (81.1%)<br>284 - 292°C (decomposition), 3300, 1650, 1570 $cm^{-1}$ |
| 16 | $OC_3H_7$(3) | H | Zinc acetate | Compound 325 (63.0%)<br>117.5 - 119°C, 1850, 1700, 1575 $cm^{-1}$ |
| 17 | $OC_6H_{13}$(3) | H | Zinc acetate | Compound 377 (95.3%)<br>184 - 186°C (decomposition), 1650, 1575 $cm^{-1}$ |

$^1$H-NMR (400 MHz)($CDCl_3$-$d_1$): δ0.97 (t, 3H), δ1.72 (m, 2H), δ4.03 (t, 2H), δ7.45 (dd, 1H, J = 4.4, 8.3), δ7.58 (d, 1H, J = 8.8), δ8.13 (d, 1H, J = 4.9)

(c) Synthesis of Copper 3-Propoxypicolate: Copper-bis[3-Propoxy-2-Pyridinecarboxylate-$N^1$,$O^2$]

A 0.85 g (4.7 mmol) amount of 3-propoxypicolinic acid was dissolved in 35 ml of water and stirred. To this was dropped a solution of 1.06 g (2.3 mmol) of copper acetate•dihydrate dissolved in 2 ml of water. After the dropping, the mixture was agitated at room temperature for 60 minutes. The resultant mixture was allowed to stand in a refrigerator over night, but no crystals could be obtained, so the solution was condensed under reduced pressure. The precipitated solid was recrystallized from ethanol to obtain 0.61 g of crystal.

(Yield: 61.3%)

m.p.: 180 °C (decomposition)

IR (KBr): 2967, 2882, 1651, 1580, 1456, 1345, 1250 $cm^{-1}$

| Element analysis: as $C_{18}H_{20}N_2O_6Cu \bullet 1/2H_2O$ | | | | |
|---|---|---|---|---|
| Calculated:<br>Analyzed: | C 49.94,<br>C 50.06, | H 4.89,<br>H 4.77, | N 6.47,<br>N 6.44, | Cu 14.68<br>Cu 14.70 |

Examples 2 - 17 (Synthesis Examples)

The step (c) of Synthesis Example 1 was repeated except that the reaction was performed with the picolinic acid derivatives of Table II and copper acetate•dihydrate, manganese chloride•dihydrate, or zinc acetate•dihydrate in a molar ratio of 1:about 2.05, whereby the coordinated complexes of Table II was obtained.

Example 18. Suppressing Action on Active Oxygen, Action on Production of Active Oxygen From Neutrophils

This Example illustrates the suppression of the production of active oxygen by the coordinated complexes of the present invention.

A 400 mL amount of physiological saline solution containing 0.2% glycogen was administered by drip into the abdominal cavity of a white Japanese rabbit having about 3 kg body weight without anesthesia. After 16 hours, 400 ml of sterilized physiological saline solution was administered, the cavity was massaged well, then the ascites was sampled. This was centrifuged at 100 × g to obtain a precipitate which was then suspended in a Hanks Balanced Salt Solution (HBSS) and adjusted to 5 x $10^5$ cells/mL. This was used as the polymorphonuclear leukocyte (PMNs) fraction. The action on the production of active oxygen by opsonized zymosan (OPZ) was studied as follows:

To PMNs (480 μl) were added 3,7-dihydro-6-[4-[2-(N'-(5-fluoresceinyl)thioureid)ethoxy]phenyl]-2-methylimidazo[1,2-a]pyradine-3-on•sodium salt (FCLA) (2 μM) and various concentrations of test compounds. The resulting mixture was incubated at 37°C for 10 minutes, OPZ was added in an amount of 5 μL, and then the FCLA dependent chemiluminescence was measured using a photon counter (made by BertHold of Germany) under a constant temperature of 37°C along with the elapse of time.

The effect of the suppression to production of active oxygen (suppressive rate) is shown in Table III. Table III shows that, except for compound 159, the coordinated complexes of the present invention exhibited substantially the same activity as ascorbic acid, tested as a comparative reagent, and that 1 μM of these coordinated complexes exhibit about the same activity as 0.1 U/mL of superoxide dismutase (SOD).

Table III. Suppressing Action on Production of Active Oxygen (Suppressive Rate %)

| Compound No. | Concentration (μM) | | |
|---|---|---|---|
| | 0.1 | 1.0 | 10 |
| Ascorbic acid | 20.1 | 51.7 | 86.8 |
| 1 | 30.9 | 92.0 | 99.5 |
| 2 | 33.6 | 91.2 | 98.6 |
| 3 | 36.8 | 88.2 | 98.2 |
| 4 | 33.9 | 92.9 | Insoluble |
| 5 | 26.0 | 85.4 | 99.6 |
| 6 | 35.1 | 88.4 | Insoluble |
| 11 | 27.8 | 89.4 | 99.6 |
| 45 | 23.3 | 88.2 | 98.9 |
| 48 | 31.0 | 84.1 | 99.5 |
| 63 | 27.7 | 92.1 | 99.8 |
| 94 | 23.7 | 85.1 | 99.2 |
| 99 | 24.2 | 79.2 | Insoluble |
| 100 | 35.9 | 90.2 | Insoluble |
| 157 | -13.9 | 12.4 | 63.1 |

Note) SOD Suppressive Rate (%)

| SOD (Concentration) | | Suppressive rate |
|---|---|---|
| 0.084 nM | 0.01 U/ml | 25.1 |
| 0.84 nM | 0.1 U/ml | 72.0 |
| 8.4 nM | 1.0 U/ml | 93.9 |

As explained above, the present invention provides active oxygen suppressive agents comprising coordinated complexes of Cu, Mn, or Zn bonded with picolinic acid derivatives as ligands. The majority of the coordinated complexes are novel complexes. Further, these novel coordinated complexes are simultaneously provided. As a specific application of the active oxygen suppressive agents, the present invention is useful as a component of a cold perfusion storage solution for organs to be transplanted.

INDUSTRIAL APPLICABILITY

The coordinated complexes according to the present invention are capable of preventing various type of disorders due to active oxygen present outside of the body and in particular can prevent cell disorders due to active oxygen in preservative solutions for organs and can eliminate the active oxygen in the body or

suppress the production of active oxygen therein. As diseases which may arise due to active oxygen in the body, there are known inflammation, various ischemic diseases, radiation disease, ageing, cataracts, autoimmune disorders, etc.

Accordingly, the active oxygen suppressive compositions of the present invention are suitable for use in the preservation solutions for transplantation of organs and also may be used as an antiphlogistic, an agent against angina pectoris, a cocomittant agent at the time of reirrigation treatment due to percutaneous transluminal coronary angioplasty (PTCA) method and intracoronary thrombolysis (ICT), agents against cerebral infarction, radiophylaxis agents, cataract treatment agents, and treatment agents for various autoimmune diseases.

## Claims

**1.** An active oxygen suppressive composition comprising, as an effective ingredient, a coordinated complex having the formula (I) and a carrier

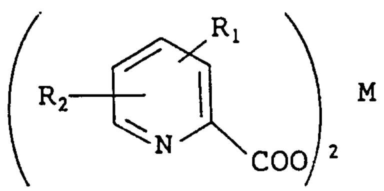

(I)

wherein, $R_1$ and $R_2$ independently represent a hydrogen atom, an alkyl group having 1 to 12 carbon atoms, an alkyloxy group having 1 to 12 carbon atoms, a halogen atom, a hydroxyl group, a nitro group, an amino group, a carboxyl group, or a lower acyloxy group having 2 to 6 carbon atoms and M represents a center atom selected from Cu, Mn, and Zn.

**2.** A coordinated complex having the formula (II):

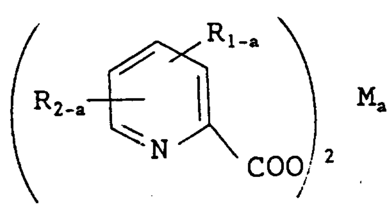

(II)

wherein, $R_{1-a}$ and $R_{2-a}$ have the same definitions as $R_1$ and $R_2$ of the formula (I) and $M_a$ is Cu or Mn, provided that when $M_a$ is Cu, $R_{1-a}$ and $R_{2-a}$ are not simultaneously a hydrogen atom, when $R_{1-a}$ is a hydrogen atom, $R_{2-a}$ is not a hydroxyl group at the 4- or 5-position, and when $M_a$ isMn, $R_{1-a}$ and $R_{2-a}$ are not simultaneously a hydrogen atom.

**INTERNATIONAL SEARCH REPORT**

International application No.
PCT/JP94/00067

A. CLASSIFICATION OF SUBJECT MATTER

Int. $Cl^5$ C07F1/08, C07F13/00, A01N1/02

According to International Patent Classification (IPC) or to both national classification and IPC

B. FIELDS SEARCHED

Minimum documentation searched (classification system followed by classification symbols)

Int. $Cl^5$ C07F1/08, C07F3/06, C07F13/00, A01N1/02, C07D213/79

Documentation searched other than minimum documentation to the extent that such documents are included in the fields searched

Electronic data base consulted during the international search (name of data base and, where practicable, search terms used)

CAS ONLINE

C. DOCUMENTS CONSIDERED TO BE RELEVANT

| Category* | Citation of document, with indication, where appropriate, of the relevant passages | Relevant to claim No. |
|---|---|---|
| X | Chemical Abstracts, 62, 9099h-9100c (1965) | 2 |
| X | Chemical Abstracts, 63, 8311c-8311e (1965) | 2 |
| X | Chemical Abstracts, 65, 758b-758c (1966) | 2 |
| A | JP, A, 56-48245 (Hidetoshi Tsuchida), May 1, 1981 (01. 05. 81), (Family: none) | 1 |

☐ Further documents are listed in the continuation of Box C. ☐ See patent family annex.

* Special categories of cited documents:

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| April 11, 1994 (11. 04. 94) | May 10, 1994 (10. 05. 94) |
| Name and mailing address of the ISA/ Japanese Patent Office | Authorized officer |
| Facsimile No. | Telephone No. |